# EUROPEAN PATENT SPECIFICATION

(11) **EP 2 653 477 B1**
(45) Date of publication and mention of the grant of the patent: **24.10.2018**
(21) Application number: 11847902.1
(22) Date of filing: 08.11.2011
(51) Int. Cl.: C07K 14/435, C12N 15/12, C12N 15/63, C12N 15/09, C12P 21/02, A61K 38/17, A61P 37/02

(54) **SCORPION ACTIVE PEPTIDES AND PREPARATION METHODS AND APPLICATIONS THEREOF**
AKTIVE SKORPIONPEPTIDE UND HERSTELLUNGSVERFAHREN SOWIE ANWENDUNGEN DAVON
PEPTIDES ACTIFS DE SCORPION ET LEURS PROCÉDÉS DE SYNTHÈSE ET APPLICATIONS

(30) Priority: 14.12.2010 CN 201010588272
(43) Date of publication of application: 23.10.2013
(73) Proprietor: Wuhan More Biotechnology Co., Ltd., Wuhan, Hubei 430079 (CN)
(72) Inventor: LI, Wenxin, Wuhan Hubei 430030 (CN); WU, Yingliang, Wuhan Hubei 430030 (CN); CAO, Zhijian, Wuhan Hubei 430030 (CN); HAN, Song, Wuhan Hubei 430030 (CN)
(74) Representative: Potter Clarkson LLP
(86) International application number: PCT/CN2011/081920
(87) International publication number: WO 2012/079435

(56) References cited:
- WO-A2-2006/002850
- WO-A2-2006/002850
- CN-A- 101 422 599
- CN-A- 101 422 600
- CN-A- 102 127 160
- CN-A- 102 127 160
- JEAN-GUILLAUME RENISIO ET AL: "Solution structure of BmKTX, a K+ blocker toxin from the Chinese scorpionButhus Martensi", PROTEINS: STRUCTURE, FUNCTION, AND BIOINFORMATICS, vol. 38, no. 1, 1 January 2000 (2000-01-01), pages 70-78, XP55071950, ISSN: 0887-3585, DOI: 10.1002/(SICI)1097-0134(20000101)38:1<70:: AID-PROT8>3.0.CO;2-5
- S. HAN ET AL: "Structural Basis of a Potent Peptide Inhibitor Designed for Kv1.3 Channel, a Therapeutic Target of Autoimmune Disease", JOURNAL OF BIOLOGICAL CHEMISTRY, vol. 283, no. 27, 14 May 2008 (2008-05-14) , pages 19058-19065, XP55111074, ISSN: 0021-9258, DOI: 10.1074/jbc.M802054200
- KOO G C ET AL: "Blockade of the voltage-gated potassium channel Kv1.3 inhibits immune responses in vivo.", JOURNAL OF IMMUNOLOGY (BALTIMORE, MD. : 1950) 1 JUN 1997, vol. 158, no. 11, 1 June 1997 (1997-06-01) , pages 5120-5128, XP002727345, ISSN: 0022-1767
- HAN, S. ET AL.: 'ImKTx88, a novel selective Kvl.3 channel blocker derived from the scorpion Isometrus maculates.' TOXICON. vol. 57, 29 December 2010, pages 348 - 355, XP028135912
- RENISIO, J.G. ET AL.: 'Solution Structure of BmKTX, a K' Blocker Toxin From the Chinese Scorpion Buthus Martensi.' PROTEINS. vol. 38, 01 January 2000, pages 70 - 78, XP055071950
- HAN, S. ET AL.: 'Structural Basis of a Potent Peptide Inhibitor Designed for Kvl.3 Channel' THERAPEUTIC TARGET OF AUTOIMMUNE DISEASE. vol. 283, no. 27, 14 May 2008, pages 19058 - 19065, XP055111074

### SEQUENCE LISTING

<110> Wuhan More Biotechnology Co., LTD.
<120> SCORPION ACTIVE PEPTIDES AND PREPARATION METHODS AND APPLICATIONS THEREOF
<130> OP111036
<160> 20
<170> PatentIn version 3.3
<210> 1
   <211> 38
   <212> PRT
   <213> Artificial sequence
<400> 1
   Gln Ile Tyr Thr Ser Lys Glu Cys Asn Gly Ser Ser Glu Cys Tyr Ser
   1               5                   10                  15
   His Cys Glu Gly Ile Thr Gly Lys Arg Ser Gly Lys Cys Ile Asn Lys
               20                  25                  30
   Lys Cys Val Cys Tyr Arg
           35
<210> 2
   <211> 37
   <212> PRT
   <213> Artificial sequence
<400> 2
   Val Gly Ile Asn Val Gln Cys Asn Thr Ser Gly Gln Cys Leu Lys Pro
   1               5                   10                  15
   Cys Lys Asp Ala Gly Met Arg Phe Gly Gln Cys Ile Asn Gly Lys Cys
               20                  25                  30
   Asp Cys Thr Pro Lys
           35
<210> 3
   <211> 37
   <212> PRT
   <213> Artificial sequence
<400> 3
   Val Gly Ile Asn Val Gln Cys Asn Gly Ser Arg Gln Cys Leu Lys Pro
   1               5                   10                  15
   Cys Lys Asp Ala Gly Met Arg Phe Gly Gln Cys Ile Asn Gly Thr Cys
                   20                  25                  30
   Asp Cys Thr Pro Lys
           35
<210> 4
   <211> 117
   <212> DNA
   <213> Artificial sequence
<400> 4
   cagatatata caagtaaaga atgtaatggg tcatcagaat gctactcaca ttgcgagggc     60
   attacaggaa aaagaagcgg caaatgcatt aacaaaaaat gtgtgtgtta cagataa       117
<210> 5
   <211> 114
   <212> DNA
   <213> Artificial sequence
<400> 5
   gtgggaataa atgtgcaatg taatacatct ggtcaatgtt tgaaaccatg caaggatgct     60
   ggaatgagat ttggacaatg cataaatggc aaatgcgact gtacaccaaa gtga          114
<210> 6
   <211> 114
   <212> DNA
   <213> Artificial sequence
<400> 6
   gtgggaataa atgtgcaatg taatggatct agacaatgtt tgaaaccatg caaggatgct     60
   ggaatgagat ttggacaatg cataaatggc acatgcgact gtacaccaaa gtga          114
<210> 7
   <211> 37
   <212> DNA
   <213> Artificial sequence
<400> 7
   gtgaattcga tgacgatgac aagcagatat atacaag 37
<210> 8
   <211> 24
   <212> DNA
   <213> Artificial sequence
<400> 8
   tagctcgagt tatctgtaac acac 24
<210> 9
   <211> 38
   <212> DNA
   <213> Artificial sequence
<400> 9
   gtgaattcga tgacgatgac aaggtgggaa taaatgtg 38
<210> 10
   <211> 25
   <212> DNA
   <213> Artificial sequence
<400> 10
   tagctcgagt cactttggtg tacag 25
<210> 11
   <211> 43
   <212> DNA
   <213> Artificial sequence
<400> 11
   gtgaattcga tgacgatgac aaggtgggaa taaatgtgca atg 43
<210> 12
   <211> 29
   <212> DNA
   <213> Artificial sequence
<400> 12
   tagctcgagt cactttggtg tacagtcgc 29
<210> 13
   <211> 37
   <212> PRT
   <213> Artificial sequence
<400> 13
   Val Gly Ile Asn Val Gln Cys Asn His Ser Gly Gln Cys Leu Lys Pro
   1               5                   10                  15
   Cys Lys Asp Ala Gly Met Arg Phe Gly Gln Cys Ile Asn Gly Lys Cys
               20                  25                  30
   Asp Cys Thr Pro Lys
           35
<210> 14
   <211> 37
   <212> PRT
   <213> Artificial sequence
<400> 14
   Val Gly Ile Asn Val Gln Cys Asn His Ser Arg Gln Cys Leu Lys Pro
   1               5                   10                  15
   Cys Lys Asp Ala Gly Met Arg Phe Gly Gln Cys Ile Asn Gly Thr Cys
               20                  25                  30
   Asp Cys Thr Pro Lys
           35
<210> 15
   <211> 114
   <212> DNA
   <213> Artificial sequence
<400> 15
   gttggaatca atgtgcaatg taatcattct ggtcaatgtt tgaaaccatg caaggatgct     60
   ggaatgagat ttggacaatg cataaatggc aaatgcgact gtacacctaa gtga          114
<210> 16
   <211> 114
   <212> DNA
   <213> Artificial sequence
<400> 16
   gtgggcatta atgtgcaatg taatcattct cggcaatgtt tgaaaccatg caaggatgct     60
   ggaatgagat ttggacaatg cataaatggc acatgcgact gtactccaaa gtga          114
<210> 17
   <211> 38
   <212> DNA
   <213> Artificial sequence
<400> 17
   gtgaattcga tgacgatgac aaggttggaa tcaatgtg 38
<210> 18
   <211> 23
   <212> DNA
   <213> Artificial sequence
<400> 18
   tagctcgagt cacttaggtg tac 23
<210> 19
   <211> 39
   <212> DNA
   <213> Artificial sequence
<400> 19
   gtgaattcga tgacgatgac aaggtgggca ttaatgtgc 39
<210> 20
   <211> 25
   <212> DNA
   <213> Artificial sequence
<400> 20
   tagctcgagt cactttggag tacag 25

## Description

This application claims the priority of China Patent Application No. 201010588272.x, filed with the Patent Office of China on December 14, 2010, titled "SCORPION ACTIVE PEPTIDES AND PREPARATION METHODS AND APPLICATIONS THEREOF".

### FIELD OF THE INVENTION

The present invention relates to the field of biological technology, specifically to scorpion active polypeptide and preparation methods and application thereof.

### BACKGROUND OF THE INVENTION

Autoimmune disease is a disease caused by the immune response of the body against the autologous antigen, which leads to the tissue damage of one's own, i.e., the immune system of the human body attacks the tissues of one's own. About 5∼8% of the world population are threatened by more than about 40 autoimmune diseases, including rheumatoid arthritis mediated by T cells, multiple sclerosis, systemic lupus erythematosus, Behcet's disease, autoimmune thyroid disease, and type I diabetes, etc. In the rheumatoid arthritis, the immune system attacks the joints of a patient, and in the multiple sclerosis, it is the myelin sheaths of nerve cells that are attacked. These diseases involve one or more tissues and organs all over the body, and severely affect the human health and life. Currently, there is no effective therapy for autoimmune diseases, and the patient's condition is recurrent. Currently, the methods against autoimmune diseases commonly used are: one is to slow down the inflammatory responses, which are caused by attacking the tissues by the immune system, using steroids; the other is to suppress the effects of the immune system using immunosuppressive drugs. However, both methods will cause severe side effects, and both can only slow down the progression speed of the patient's condition, but can not eradicate the disease. In order to change the backward situation of the pharmacotherapy for autoimmune diseases, there is an urgent need to explore new methods for prognosing and treating such diseases.

After the naïve T cells are activated by specific antigenic substance, they proliferate and differentiate, and generally form two types of functionally different cells, i.e. effector memory T (TEM) cell and central memory T (TCM) cell. There exists two types of potassium channels, one type is voltage-gated calcium channel Kv1.3, the other type is intermediate-conductance calcium-activated potassium channel (IKCa1). In recent years, studies have shown that there are significant spatial and temporal expression differences of potassium channel Kv1.3 in different types of T cells. After T cells are activated, only the TEM cells with immune functions can significantly express 1500∼2000 Kv1.3 potassium channels. Such conditions of high expression of potassium channel Kv1.3 have been confirmed successively in TEM cells of patients suffering from different autoimmune diseases. Therefore, potassium channel Kv1.3 on the T cell membrane holds new therapeutic promise for autoimmune diseases [J. Clin. Invest., 2003, 111: 1703; Immunity, 2008, 29: 602].

Organic small molecule drugs have initially received more attention. For example, Merck & Co., Valter and Eliza Hall Institute of Medical Research in Australia, University of California in the US, and other institutions have screened, isolated, synthesized, reconstructed, and identified plenty of organic small molecule drug candidates [Mol. Pharmacol, 2004, 65:1364; Journal of Medicinal Chemistry, 2006, 49(4), 1433; Mol. Pharmacol, 2005, 68:1254]. However, organic small molecule candidate drugs almost all act on potassium Kv1.3 and other similar potassium channels, and thus will likely cause potent toxic and side effects, for example, the activities of the organic small molecule drugs acting on homologous channels of Kv1.1, Kv1.2, Kvl.4, Kv1.5, etc. are 2∼70 times lower than that on the Kvl.3 channels. Due to the huge challenge for screening and designing organic small molecule candidate drugs, the screening and designing of polypeptides will become a new trend.

In traditional Chinese medicines, the strategy of "cure a poisoned patient with poison" is commonly used in the treatment of difficult miscellaneous diseases in human, and toxic animals such as scorpion, snake, spider, toad, centipede, etc. are the preferred. Modern studies have shown that the venoms of these toxic animals are rich in animal active polypeptides, and these polypeptides can specifically act on ion channels on the cell membrane. Nowadays, these animal active polypeptides have become important drug resources which are globally competed for their research and development [Nat. Rev. Drug. Discov. 2003 2: 63].

Koo G C et al, Journal of Immunology, 1 Jun 1997, Vol. 158, no. 11, pg 5120-5128 describes in vitro studies which indicate that blockage of Kv1.3 inhibits T cell activation, suggesting that Kv1.3 may be a target for immunosuppression.

### SUMMARY OF THE INVENTION

The purpose of the present invention is to provide a stable scorpion active polypeptide for the treatment of autoimmune diseases.

To achieve the purpose of the present invention, the present invention employs the following technical solutions:

In the present invention, many polypeptide genes having important pharmaceutical prospects are obtained by screening the gene library of scorpion active polypeptide in our country, and 3 new genes of scorpion active peptide which can specifically act on potassium channel Kvl.3 have been screened out by virtual screening, through the established computer-aided screening and designing techniques [Biophysical Journal, 2004, 87: 105; Proteins, 2008, 70: 744; Journal of Proteome Research, 2010, 9: 3118]. Three scorpion active polypeptides are obtained by genetic engineering techniques, these three scorpion active polypeptide molecules all contain 3 pairs of disulfide bonds, and thus have strong *in vitro* stabilities, and are easy for long-term storage. According to the structural characteristics of scorpion active polypeptide in taxonomy, these three all belong to the same subtype of α-KTX family, and they all act on the potassium channel Kv1.3.

In one embodiment, the present invention provides a scorpion active peptide having the amino acid sequence as set forth in SEQ ID NO: 1, which is named ADMR-011.

In one embodiment, the present invention provides a scorpion active polypeptide having the amino acid sequence as set forth in SEQ ID NO: 2, which is named ADMR-016.

In one embodiment, the present invention provides a scorpion active polypeptide having the amino acid sequence as set forth in SEQ ID NO: 3, which is named ADMR-019.

In one embodiment, the present invention provides a scorpion active peptide having the amino acid sequence as set forth in SEQ ID NO: 13, which is named ADMR-023; in another embodiment, the present invention provides a scorpion active polypeptide having the amino acid sequence as set forth in SEQ ID NO: 14, which is named ADMR-028.

A polypeptide having an amino acid sequence obtained by deleting or adding one or more amino acids in the amino acid sequences as set forth in SEQ ID NO: 1, SEQ ID NO: 2, SEQ ID NO: 3, SEQ ID NO: 13 or SEQ ID NO: 14 is here disclosed.

The present invention further provides DNA molecules that encode the scorpion active polypeptides ADMR-011, ADMR-016, ADMR-019, ADMR-023 and ADMR-028 according to the present invention. Due to the degeneracy of codons, there may exist a plurality of nucleotide sequences that can encode the specific polypeptide according to the present invention.

In one embodiment, the present invention provides a DNA molecule that can encode the scorpion active polypeptide ADMR-011 having the amino acid sequence as set forth in SEQ ID NO: 1, and the nucleotide sequence thereof is as set forth in SEQ ID NO: 4.

In one embodiment, the present invention provides a DNA molecule that can encode the scorpion active polypeptide ADMR-016 having the amino acid sequence as set forth in SEQ ID NO: 2, and the nucleotide sequence thereof is as set forth in SEQ ID NO: 5.

In another embodiment, the present invention provides a DNA molecule that can encode the scorpion active polypeptide ADMR-019 having the amino acid sequence as set forth in SEQ ID NO: 3, and the nucleotide sequence thereof is as set forth in SEQ ID NO: 6.

In one embodiment, the present invention provides a DNA molecule that can encode the scorpion active polypeptide ADMR-023 having the amino acid sequence as set forth in SEQ ID NO: 13, and the nucleotide sequence thereof is as set forth in SEQ ID NO: 15.

In another embodiment, the present invention provides a DNA molecule that can encode the scorpion active polypeptide ADMR-028 having the amino acid sequence as set forth in SEQ ID NO: 14, and the nucleotide sequence thereof is as set forth in SEQ ID NO: 16.

Meanwhile, the present invention provides a vector having a DNA molecule that encodes the scorpion active polypeptide ADMR-011, ADMR-016, ADMR-019, ADMR-023 or ADMR-028 according to the present invention.

In one embodiment, the present invention provides a vector having a DNA molecule that can encode the scorpion active polypeptide ADMR-011 according to the present invention. Wherein, as a preference, the vector is pGEX-6p-1 having a DNA molecule that can encode the scorpion active polypeptide ADMR-011. More preferably, the vector is pGEX-6p-1 having the nucleotide sequence as set forth in SEQ ID NO: 4.

In one embodiment, the present invention provides a vector having a DNA molecule that can encode the scorpion active polypeptide ADMR-016 according to the present invention. Wherein, as a preference, the vector is pGEX-6p-1 having a DNA molecule that can encode the scorpion active polypeptide ADMR-016. More preferably, the vector is pGEX-6p-1 having the nucleotide sequence as set forth in SEQ ID NO: 5.

In another embodiment, the present invention provides a vector having a DNA molecule that can encode the scorpion active polypeptide ADMR-019 according to the present invention. Wherein, as a preference, the vector is pGEX-6p-1 having a DNA molecule that can encode the scorpion active polypeptide ADMR-019. More preferably, the vector is pGEX-6p-1 having the nucleotide sequence as set forth in SEQ ID NO: 6.

In one embodiment, the present invention provides a vector having a DNA molecule that can encode the scorpion active polypeptide ADMR-023 according to the present invention. Wherein, as a preference, the vector is pGEX-6p-1 having a DNA molecule that can encode the scorpion active polypeptide ADMR-023. More preferably, the vector is pGEX-6p-1 having the nucleotide sequence as set forth in SEQ ID NO: 15.

In another embodiment, the present invention provides a vector having a DNA molecule that can encode the scorpion active polypeptide ADMR-028 according to the present invention. Wherein, as a preference, the vector is pGEX-6p-1 having a DNA molecule that can encode the scorpion active polypeptide ADMR-028. More preferably, the vector is pGEX-6p-1 having the nucleotide sequence as set forth in SEQ ID NO: 16.

The present invention further provides a method for preparing the scorpion active polypeptide ADMR-011, ADMR-016, ADMR-019, ADMR-023 or ADMR-028, comprising:
step 1: obtaining DNA a molecule that can encode the amino acid sequence as set forth in SEQ ID NO: 1, SEQ ID NO: 2, SEQ ID NO: 3, SEQ ID NO: 13 or SEQ ID NO: 14;
step 2: fusing the DNA molecule obtained in step 1 with an expression vector, and constructing a recombinant expression vector, and transforming a host cell;
step 3: inducing the host cell containing the recombinant expression vector to express the protein, and isolating and purifying the expressed protein.

Currently, there are many methods for obtaining DNA molecules using genetic engineering techniques, including digesting the vector having the DNA molecule that encodes the scorpion active polypeptide by using restriction endonuclease, or performing PCR amplification by using cDNA having the DNA molecule that encodes the scorpion active polypeptide as templates.

In one embodiment, in the method for preparing the scorpion polypeptide ADMR-011 according to the present invention, step 1 is specifically carried out by an amplification using a template of cDNA encoding the amino acid sequence as set forth in SEQ ID NO: 1, and an upstream primer having the nucleotide sequence as set forth in SEQ ID NO: 7 and a downstream primer having the nucleotide sequence as set forth in SEQ ID NO: 8.

In one embodiment, in the method for preparing the scorpion polypeptide ADMR-016 according to the present invention, step 1 is specifically carried out by an amplification using a template of cDNA encoding the amino acid sequence as set forth in SEQ ID NO: 2, and an upstream primer having the nucleotide sequence as set forth in SEQ ID NO: 9 and a downstream primer having the nucleotide sequence as set forth in SEQ ID NO: 10.

In another embodiment, in the method for preparing the scorpion polypeptide ADMR-019 according to the present invention, step 1 is specifically carried out by an amplification using a template of cDNA encoding the amino acid sequence as set forth in SEQ ID NO: 3, and an upstream primer having the nucleotide sequence as set forth in SEQ ID NO: 11 and a downstream primer having the nucleotide sequence as set forth in SEQ ID NO: 12.

In one embodiment, in the method for preparing the scorpion polypeptide ADMR-023 according to the present invention, step 1 is specifically carried out by an amplification using a template of cDNA encoding the amino acid sequence as set forth in SEQ ID NO: 13, and an upstream primer having the nucleotide sequence as set forth in SEQ ID NO: 17 and a downstream primer having the nucleotide sequence as set forth in SEQ ID NO: 18.

In another embodiment, in the method for preparing the scorpion polypeptide ADMR-028 according to the present invention, step 1 is specifically carried out by an amplification using a template of cDNA encoding the amino acid sequence as set forth in SEQ ID NO: 14, and an upstream primer having the nucleotide sequence as set forth in SEQ ID NO: 19 and a downstream primer having the nucleotide sequence as set forth in SEQ ID NO: 20.

The expression vector of step 2 in the preparation method according to the present invention includes pGEX series, pET series, pQE series and pMAL series. In a preferred embodiment, the expression vector is pGEX-6p-1 in pGEX series.

The host cell of step 2 in the preparation method according to the present invention is an expression strain of *E. coli,* including strains in Rosetta series and BL21 series. In a preferred embodiment, the host cell is of Rosetta (DE3) strain.

Experiments have shown that the scorpion active polypeptides ADMR-011, ADMR-016, ADMR-019, ADMR-023 and ADMR-028 according to the present invention can specifically act on potassium channel Kv1.3, and can effectively treat autoimmune diseases of multiple sclerosis and rheumatoid arthritis, etc. in rat animal level. Therefore, the present invention further provides use of the scorpion polypeptide in the manufacture of a medicament for treating and diagnosing a disease associated with potassium channel Kvl.3, the disease associated with potassium channel Kv1.3 includes autoimmune diseases, specifically multiple sclerosis or rheumatoid arthritis.

### DESCRIPTION OF THE DRAWINGS

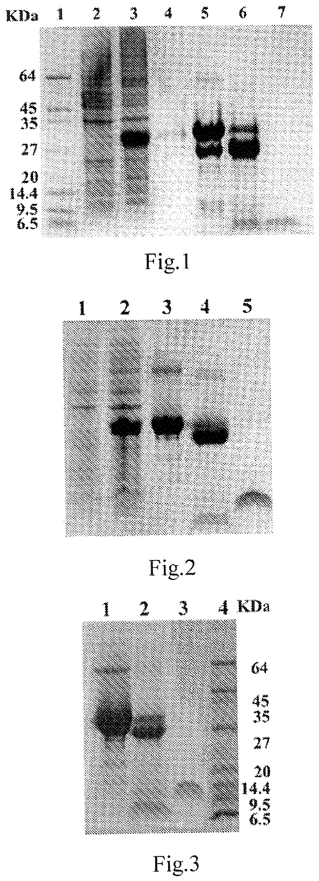

Figure 1 shows the electrophoretogram of the recombinant scorpion active polypeptide ADMR-011 and the GST fusion expressed protein thereof, wherein, lane 1 is the marker of protein molecular weight; lane 2 is the ADMR-011 in whole cell proteins without IPTG induction; lane 3 is the ADMR-011 in whole cell proteins with IPTG induction; lane 4 is the fusion protein of GST-ADMR-011 after affinity chromatography; lane 5 is the fusion protein of GST-ADMR-011 after ultrafiltration, desalination, and concentration; lane 6 is the EK digested product of GST-ADMR-011; and lane 7 is the ADMR-011 polypeptide by HPLC isolation and purification;
Figure 2 shows the electrophoretogram of the recombinant scorpion active polypeptide ADMR-016 and the GST fusion expressed protein thereof, wherein, lane 1 is the ADMR-016 in whole cell proteins without IPTG induction; lane 2 is the ADMR-016 in whole cell proteins with IPTG induction; lane 3 is the fusion protein of GST-ADMR-016 after ultrafiltration, desalination, and concentration; lane 4 is the EK digested product of GST-ADMR-016; and lane 5 is the ADMR-016 polypeptide by HPLC isolation and purification;
Figure 3 shows the electrophoretogram of the recombinant scorpion active polypeptide ADMR-019 and the GST fusion expressed protein thereof, wherein, lane 1 is the fusion protein of GST-ADMR-019 after ultrafiltration, desalination, and concentration; lane 2 is the EK digested product of GST-ADMR-019; and lane 3 is the ADMR-019 polypeptide by HPLC isolation and purification; and lane 4 is the marker of protein molecular weight;

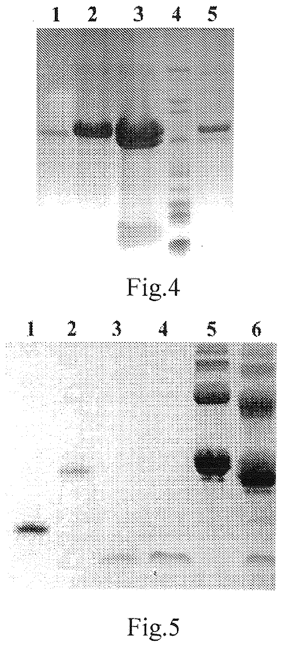

Figure 4 shows the electrophoretogram of the recombinant scorpion active polypeptide ADMR-023 and the GST fusion expressed protein thereof, wherein, 1 is the fusion protein GST-ADMR-023 obtained by affinity chromatography purification; 2 is the fusion protein of GST-ADMR-023 after ultrafiltration, desalination, and concentration; 3 is the EK digested product of GST-ADMR-023; 4 is marker of low range molecular weight (the molecular weights from high to low are 64 KD, 45 KD, 35 KD, 27 KD, 20 KD, 14.4 KD, 9.5 KD, 6.5 KD and 4 KD, respectively); and 5 is the fusion protein GST-ADMR-023 obtained by affinity chromatography.
Figure 5 shows the electrophoretogram of the recombinant scorpion active peptide ADMR-028 and the GST fusion expressed protein thereof.
   1 is lysozyme; 2 is fusion protein GST-ADMR-028 obtained by affinity chromatography purification; 3 is mutant polypeptide ADMR-028 obtained by HPLC isolation and purification; 4 is mutant polypeptide ADMR-028 obtained by HPLC isolation and purification; 5 is fusion protein GST-ADMR-028 after ultrafiltration, desalination, and concentration; and 6 is the EK digested product of GST-ADMR-028.
Figure 6 shows the chromatogram for isolating and purifying the recombinant scorpion active polypeptide ADMR-011 and GST protein;
Figure 7 shows the chromatogram for isolating and purifying the recombinant scorpion active polypeptide ADMR-016 and GST protein;
Figure 8 shows the chromatogram for isolating and purifying the recombinant scorpion active polypeptide ADMR-019 and GST protein;
Figure 9 shows the chromatogram for isolating and purifying the recombinant scorpion active polypeptide ADMR-023 and GST protein;
Figure 10 shows the chromatogram for isolating and purifying the recombinant scorpion active polypeptide ADMR-028 and GST protein;
Figure 11 shows the mass spectrogram of recombinant scorpion active polypeptide ADMR-011;
Figure 12 shows the mass spectrogram of recombinant scorpion active polypeptide ADMR-016;
Figure 13 shows the mass spectrogram of recombinant scorpion active polypeptide ADMR-019;
Figure 14 shows the mass spectrogram of recombinant scorpion active polypeptide ADMR-023;
Figure 15 shows the mass spectrogram of recombinant scorpion active polypeptide ADMR-028;
Figure 16 shows the schematic diagram of current suppression on potassium channel Kv1.3 by 100 pM scorpion active polypeptide ADMR-011;
Figure 17 shows the schematic diagram of current suppression on potassium channel Kv1.3 by 1 nM scorpion active polypeptide ADMR-016;
Figure 18 shows the schematic diagram of current suppression on potassium channel Kv1.3 by 1 nM scorpion active polypeptide ADMR-019;
Figure 19 shows the schematic diagram of current suppression on potassium channel Kvl.3 by 1 nM scorpion active polypeptide ADMR-023;
Figure 20 shows the schematic diagram of current suppression on potassium channel Kv1.3 by 1 nM scorpion active polypeptide ADMR-028.

### DETAILED EMBODIMENTS

In order to further understand the present invention, the preferred embodiments of the present invention are described below in conjunction with examples. It should be understood, however, these descriptions are only for further illustrating the characteristics and advantages of the present invention, but not for limiting the claims of the present invention.

According to the present invention, three novel scorpion active polypeptides are obtained by screening the gene library of scorpion active polypeptides in our country, using genetic engineering techniques. All the three peptides have 3 pairs of disulfide bonds in their molecules, and thus have strong *in vitro* stabilities, and are easy for long-term storage. In one embodiment, the present invention provides a scorpion active polypeptide, named ADMR-011, having the amino acid sequence as set forth in SEQ ID NO: 1. In one embodiment, the present invention provides a scorpion active polypeptide, named ADMR-016, having the amino acid sequence as set forth in SEQ ID NO: 2. In another embodiment, the present invention provides a scorpion active polypeptide, named ADMR-019, having the amino acid sequence as set forth in SEQ ID NO: 3. In one embodiment, the present invention provides scorpion active polypeptides, named ADMR-023 and ADMR-28 respectively, having the amino acid sequences as set forth in SEQ ID NO: 13 and SEQ ID NO: 14.

A polypeptide having an amino acid sequence obtained by deleting or adding one or more amino acids in the amino acid sequences as set forth in SEQ ID NO: 1, SEQ ID NO: 2, SEQ ID NO: 3, SEQ ID NO: 13 or SEQ ID NO: 14 is here disclosed.

The present invention further provides a DNA molecule that encodes the scorpion active polypeptides ADMR-011, ADMR-016, ADMR-019, ADMR-023 or ADMR-028 according to the present invention.

In one embodiment, the present invention provides a DNA molecule that can encode the scorpion active polypeptide ADMR-011, and the nucleotide sequence thereof is as set forth in SEQ ID NO: 4.

In one embodiment, the present invention provides a DNA molecule that can encode the scorpion active polypeptide ADMR-016, and the nucleotide sequence thereof is as set forth in SEQ ID NO: 5.

In another embodiment, the present invention provides a DNA molecule that can encode the scorpion active polypeptide ADMR-019, and the nucleotide sequence thereof is as set forth in SEQ ID NO: 6.

In one embodiment, the present invention provides a DNA molecule that can encode the scorpion active polypeptides ADMR-023 and ADMR-028, and the nucleotide sequences thereof are as set forth in SEQ ID NO: 15 and SEQ ID NO: 16.

Meanwhile, the present invention provides vectors having DNA molecules that encode the scorpion active polypeptides ADMR-011, ADMR-016, ADMR-019, ADMR-023 and ADMR-028 according to the present invention.

In one embodiment, the present invention provides a vector having a DNA molecule that can encode the scorpion active polypeptide ADMR-011 according to the present invention. Wherein, in one embodiment, the vector is pGEX-6p-1 having a DNA molecule that can encode the scorpion active polypeptide ADMR-011. More preferably, the vector is pGEX-6p-1 having the nucleotide sequence as set forth in SEQ ID NO: 4.

In one embodiment, the present invention provides a vector having a DNA molecule that can encode the scorpion active polypeptide ADMR-016 according to the present invention. Wherein, in one embodiment, the vector is pGEX-6p-1 having a DNA molecule that can encode the scorpion active polypeptide ADMR-016. More preferably, the vector is pGEX-6p-1 having the nucleotide sequence as set forth in SEQ ID NO: 5.

In one embodiment, the present invention provides a vector having a DNA molecule that can encode the scorpion active polypeptide ADMR-019 according to the present invention. Wherein, in one embodiment, the vector is pGEX-6p-1 having a DNA molecule that can encode the scorpion active polypeptide ADMR-019. More preferably, the vector is pGEX-6p-1 having the nucleotide sequence as set forth in SEQ ID NO: 6.

In one embodiment, the present invention provides a vector having a DNA molecule that can encode the scorpion active polypeptide ADMR-023 or ADMR-028 according to the present invention. Wherein, in one embodiment, the vector is pGEX-6p-1 having a DNA molecule that can encode the scorpion active polypeptide ADMR-023 or ADMR-028. More preferably, the vector is pGEX-6p-1 having the nucleotide sequence as set forth in SEQ ID NO: 15 or SEQ ID NO: 16.

In order to prepare the scorpion polypeptide according to the present invention, the present invention further provides a method for preparing the scorpion active polypeptide, comprising:
step 1: obtaining a DNA molecule that can encode the scorpion active polypeptide ADMR-011, ADMR-016, ADMR-019, ADMR-023 or ADMR-028;
step 2: fusing the DNA molecule obtained in step 1 with an expression vector, and constructing a recombinant expression vector, and transforming a host cell;
step 3: inducing the host cell containing the recombinant expression vector to express a fusion protein, and isolating and purifying the expressed fusion protein.

Currently, there are many methods for obtaining DNA molecules using genetic engineering techniques, including digesting the vector having the DNA molecule that encodes the scorpion active polypeptide using restriction endonuclease, or performing PCR amplification using cDNA having the DNA molecule that encodes the scorpion active polypeptide as template.

In one embodiment, the present invention provides an amplification using a template of cDNA encoding the scorpion active polypeptide ADMR-011, and an upstream primer having the nucleotide sequence as set forth in SEQ ID NO: 7 and a downstream primer having the nucleotide sequence as set forth in SEQ ID NO: 8, to obtain DNA molecules that encode the scorpion active polypeptide ADMR-011.

In one embodiment, the present invention provides an amplification using a template of cDNA encoding the scorpion active polypeptide ADMR-016, and an upstream primer having the nucleotide sequence as set forth in SEQ ID NO: 9 and a downstream primer having the nucleotide sequence as set forth in SEQ ID NO: 10, to obtain DNA molecules that encode the scorpion active polypeptide ADMR-016.

In another embodiment, the present invention provides an amplification using a template of cDNA encoding the scorpion active polypeptide ADMR-019, and an upstream primer having the nucleotide sequence as set forth in SEQ ID NO: 11 and a downstream primer having the nucleotide sequence as set forth in SEQ ID NO: 12, to obtain DNA molecules that encode the scorpion active polypeptide ADMR-019.

In one embodiment, the present invention provides an amplification using a template of cDNA encoding the scorpion active polypeptide ADMR-023, and an upstream primer having the nucleotide sequence as set forth in SEQ ID NO: 17 and a downstream primer having the nucleotide sequence as set forth in SEQ ID NO: 18, to obtain DNA molecules that encode the scorpion active polypeptide ADMR-023.

In another embodiment, the present invention provides an amplification using a template of cDNA encoding the scorpion active polypeptide ADMR-028, and an upstream primer having the nucleotide sequence as set forth in SEQ ID NO: 19 and a downstream primer having the nucleotide sequence as set forth in SEQ ID NO: 20, to obtain DNA molecules that encode the scorpion active polypeptide ADMR-028.

As described in step 2 in the preparation method according to the present invention, fusing the DNA molecule obtained in step 1 with an expression vector, and constructing a recombinant expression vector, and transforming a host cell. Specifically, the DNA molecule obtained in step 1 is recovered by gel electrophoresis and inserted into an expression vector by double digestion to construct recombinant expression plasmids, which are transformed into *E*. *coli.*

Wherein, the expression vector according to the present invention includes pGEX series, pET series, pQE series and pMAL series. In a preferred embodiment, the expression vector is pGEX-6p-1 in pGEX series.

The transformed host cell according to the present invention is a expression strain of *E. coli,* including strains in Rosetta series and BL21 series. In a preferred embodiment, the host cell is of Rosetta (DE3) strain. Codons preferred by eukaryotic cells are different from that of a prokaryotic cell. Therefore, when expressing eukaryotic genes with a prokaryotic system, some of the condons in eukaryotic genes can be rare codons for prokaryotic cells, resulting in low expressing efficiency and expressing level. Rosetta (DE3) is a BL21-derived bacterium carrying pRARE2 plasmid, and can supplement tRNAs corresponding to the seven rare codons (AUA, AGG, AGA, CUA, CCC, GGA and CGG), which are lacked in *E. coli,* so as to increase the expressing level of an exogenous gene, especially an eukaryotic gene, in a prokaryotic system.

Step 3 in the preparation method according to the present invention is, specifically, culturing host cells containing the recombinant vector by IPTG induction, collecting the thalli and breaking them by ultrasonication, taking the supernatant and performing affinity chromatography to obtain the fused protein, then performing desalination and concentration, digesting by enterokinase, and separating by chromatography, to obtain a chromatographically pure scorpion active polypeptide. Wherein, the chromatography medium is a matched chromatography medium selected according to the selective label of the expression vector. In a preferred embodiment, the expression vector is pGEX-6p-1, and the chromatography medium of the affinity chromatography is GST affinity chromatography gel.

In specific embodiments, the preparation method according to the present invention is specifically, obtaining a DNA molecule encoding the scorpion active polypeptide with a template of cDNA encoding the scorpion active polypeptide ADMR-011, ADMR-016, ADMR-019, ADMR-023 or ADMR-028, by using primers designed according to the nucleotide sequence that encodes the mature peptide moiety, recovering the obtained DNA molecule and expression vector pGEX-6p-1 by gel electrophoresis and double digestion, ligating the constructed recombinant expression plasmid, and transforming *E. coli* Rosetta (DE3) to obtain the recombinant bacteria. Then, culturing host cells containing the recombinant vector by IPTG induction, collecting the thalli and breaking them by ultrasonication, taking the supernatant and performing affinity chromatography to obtain the fused protein, then performing desalination and concentration, digesting by enterokinase, and separating by chromatography, to obtain a chromatographically pure scorpion active polypeptide ADMR-011, ADMR-016, ADMR-019, ADMR-023 or ADMR-028.

In the present invention, the pharmacological activities of the scorpion polypeptides on potassium channel Kvl.3 are identified by patch clamp techniques. The results show that the scorpion active polypeptides ADMR-011, ADMR-016, ADMR-019, ADMR-023 and ADMR-028 can specifically inhibit the current of potassium channel Kv1.3, and the polypeptide concentrations that inhibit the current by half (IC₅₀ value) are respectively 91 pM, 2.58 nM, 1.41 nM, and 0.49 nM, 0.65 nM, indicating that the scorpion active polypeptide ADMR-011, ADMR-016, ADMR-019, ADMR-023 or ADMR-028 can specifically act on potassium channel Kvl.3 which is a target for autoimmune disease.

In the present invention, the pharmaceutically therapeutic effects of the scorpion active polypeptides are tested by conducting simulated treatment of multiple sclerosis and rheumatoid arthritis on rat animal level. Results show that, after the treatment by ADMR-011, ADMR-016, ADMR-019, ADMR-023 and ADMR-028, the symptoms of multiple sclerosis and rheumatoid arthritis in rats are significantly improved, suggesting that the scorpion active polypeptide ADMR-011, ADMR-016, ADMR-019, ADMR-023 or ADMR-028 can effectively treat multiple sclerosis and rheumatoid arthritis.

In the present invention, it has been found, by toxicity studies on scorpion active polypeptides, that the scorpion active polypeptides ADMR-011, ADMR-016, ADMR-019, ADMR-023 and ADMR-028 have no significant toxicity to mice at a dosage 100 times more than the therapeutic dosage for the animal model of an autoimmune disease, suggesting the toxic and side effects of ADMR-011, ADMR-016, ADMR-019, ADMR-023 and ADMR-028 are low.

Therefore, the present invention further provides a use of the scorpion active polypeptide in the manufacture of a medicament for treating or preventing a disease associated with potassium channel Kvl.3, comprising autoimmune diseases, specifically multiple sclerosis or rheumatoid arthritis.

The scorpion active polypeptides ADMR-011, ADMR-016, ADMR-019, ADMR-023 and ADMR-028 according to the present invention all contain 3 pairs of disulfide bonds in their molecules, and thus have good *in vitro* stabilities, and are easy for long-term storage. Identification by patch clamp techniques shows that they can specifically act on potassium channel Kvl.3 with high specificity, and they are polypeptides with potent activity that are currently found internationally. Animal experiments show that the recombinant polypeptides ADMR-011, ADMR-016, ADMR-019, ADMR-023 and ADMR-028 can effectively treat multiple sclerosis and rheumatoid arthritis in rat with significant pharmaceutical effects and have less toxic and side effects on experimental animals. The method for preparing scorpion active polypeptide according to the present invention is simple to perform, convenient to operate, and is easy to produce and prepare the scorpion active polypeptide with high purity.

The effects of the present invention are illustrated below by detailed examples, but the protecting scope of the present invention is not limited to the following examples.

### Example 1: Obtaining of the genes of scorpion active polypeptides ADMR-011, ADMR-016, ADMR-019, ADMR-023 and ADMR-028

Scorpion tail glands of 40 isometric maculates were taken, and total RNA was extracted using Trizol reagent (Invitrogen). Refer to the kit instructions for the extraction process. mRNA was purified using Poly A Tract mRNA Isolation System (Promega) kit, and cDNA library was constructed using Superscript Plasmid System cDNA library construction kit (Gibco/BRL) kit. cDNA was cloned into pSPORT 1 vector, and transformed into *E. coli* DH5α, and cDNA clones were picked randomly for sequencing. The sequencing results were analyzed, and 236 new genes of scorpion active peptides which can interact with potassium ion channel were obtained. The spatial structures of the polypeptides encoded by the 136 scorpion active peptide genes were predicted respectively by the established computer-aided screening and designing techniques [Biophysical Journal, 2004, 87: 105; Proteins, 2008, 70: 744; Journal of Proteome Research, 2010, 9: 3118]. Then, they were screened for their interaction with potassium Kv1.3 by computerized virtual screening, and cDNA gene having a DNA molecule that encodes scorpion active polypeptide ADMR-011, ADMR-016, ADMR-019, ADMR-023 or ADMR-028 was obtained, and the encoded mature peptides were respectively the scorpion active polypeptides ADMR-011, ADMR-016, ADMR-019, ADMR-023 and ADMR-028 having the amino acid sequences as set forth in SEQ ID NO: 1, SEQ ID NO: 2, SEQ ID NO: 3, SEQ ID NO: 13 and SEQ ID NO: 14.

### Example 2: Amplification of the DNA molecule encoding the scorpion active polypeptides ADMR-011, ADMR-016, ADMR-019, ADMR-023 and ADMR-028

### 1. Primer design

Primers were designed using nucleotide sequences encoding mature peptide moiety in the cDNA gene sequence of the scorpion active polypeptides ADMR-011, ADMR-016, ADMR-019, ADMR-023 and ADMR-028, respectively. The primers designed were:
the upstream primer of ADMR-011 was the amino acid sequence as set forth in SEQ ID NO: 7;
the downstream primer of ADMR-011 was the amino acid sequence as set forth in SEQ ID NO: 8;
the upstream primer of ADMR-016 was the amino acid sequence as set forth in SEQ ID NO: 9;
the downstream primer of ADMR-016 was the amino acid sequence as set forth in SEQ ID NO: 10;
the upstream primer of ADMR-019 was the amino acid sequence as set forth in SEQ ID NO: 11;
the downstream primer of ADMR-019 was the amino acid sequence as set forth in SEQ ID NO: 12;
the upstream primer of ADMR-023 was the amino acid sequence as set forth in SEQ ID NO: 17;
the downstream primer of ADMR-023 was the amino acid sequence as set forth in SEQ ID NO: 18;
the upstream primer of ADMR-028 was the amino acid sequence as set forth in SEQ ID NO: 19;
the downstream primer of ADMR-028 was the amino acid sequence as set forth in SEQ ID NO: 20.

### 2. Amplification of DNA molecule:

DNA molecule of ADMR-011 was amplified as an example, wherein PCR amplification was performed with cDNA of scorpion venom as template, by using a upstream primer having the nucleotide sequence as set forth in SEQ ID NO: 7 and a downstream primer having the nucleotide sequence as set forth in SEQ ID NO: 8.

The reaction system for the PCR amplification was:

| | |
|---|---|
| 10×taq buffer (with 1.5 mM Mg²⁺) | 5 µl |
| dNTP (5 mM) | 4 µl |
| upstream primer (100 ng/ul) | 1 µl |
| downstream primer (100 ng/ul) | 1 µl |
| template (5∼50 ng/µl) | 1 µl |
| Taq DNA Polymerase (2 U/µl) | 0.5 µl |

sterile distilled water was added to supplement the system to a total volume of 50 µl.

PCR protocol was:

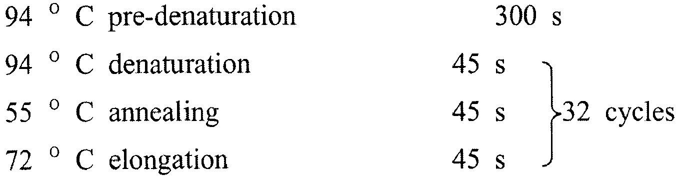

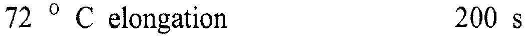

### 3. Construction of the recombinant expression vector:

The PCR amplification products were recovered by gel electrophoresis, and double digested by EcoRI and XhoI, and the digested fragments were inserted into the expression vector pGEX-6p-1 (purchased from Pharmacia Corporation) double digested by EcoRI and XhoI, to construct recombinant expression plasmids, which were transformed into *E. coli* DH5α (China Center for Type Culture Collection). Single colonies were picked from LB plates containing ampicillin, and cultured in LB liquid medium containing ampicillin at 37 ° C for 5 hours, then these cultures were identified by the a method of PCR amplification. The bacteria liquid obtained by culturing was used as template for PCR amplification, and the primers, reaction conditions, and reaction protocols were the same to that for the amplification of DNA molecule. The correct strains verified by PCR identification were sequenced, to obtain the strains containing the recombinant expression vector with the correct sequence. Then, plasmids were extracted and transformed into *E. coli* Rosetta (DE3) (China Center for Type Culture Collection).

The reaction system and reaction protocol and the construction of recombinant vector for scorpion active polypeptides ADMR-016, ADMR-019, ADMR-023 and ADMR-028 were the same to that for ADMR-011.

### Example 3. Expression and purification of the scorpion active polypeptides ADMR-011, ADMR-016, ADMR-019, ADMR-023 and ADMR-028

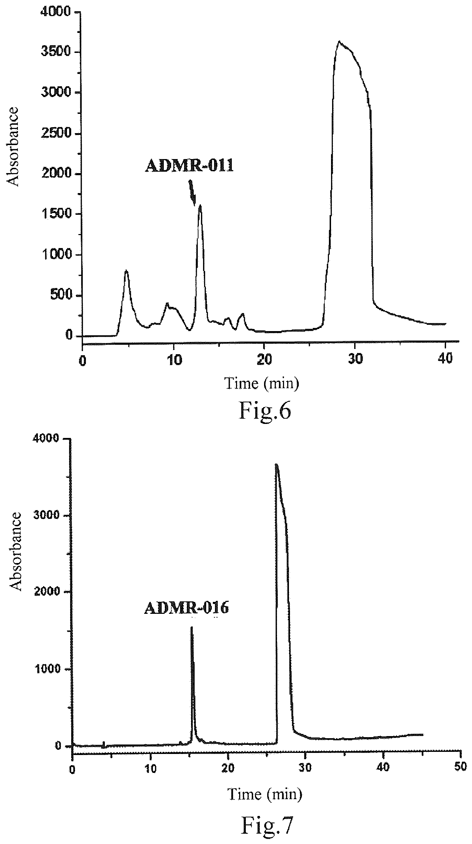

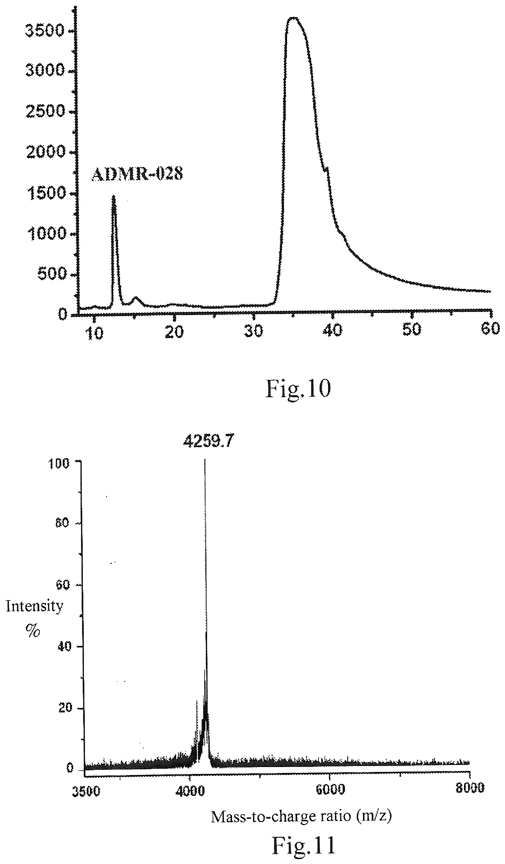

The plasmids of pGEX-6p-1-ADMR-011 strain containing recombinant expression vector with correct sequence were extracted and transformed into *E. coli* Rosetta (DE3). The transformed *E. coli* were cultured by IPTG (Sino-American biotechnology Co., LTD.) induction, and the thalli were collected, suspended in a buffer (50 mM Tris-Cl, 1.0 mM EDTA, pH 8.0), and broken by ultrasonication, and the supernatant was collected after centrifugation. The obtained supernatant was purified by GST affinity chromatography to obtain fusion protein. The obtained solution of fusion protein was desalinated and concentrated, and digested by enterokinase, to obtain the scorpion active polypeptide ADMR-011. The ADMR-011 polypeptide was further isolated by HPLC to remove GST protein, to obtain chromatographically pure ADMR-011 polypeptide. The theoretical molecular weight of ADMR-011 polypeptide was 4258.7 Da, and its molecular weight analyzed by mass spectrometry was 4258.8 Da, which was identical to the molecular weight deduced according to the amino acid sequence. Wherein, the ADMR-011 polypeptide and the GST fusion protein thereof obtained at different stages were tested by PAGE electrophoresis, and the results are shown in Figure 1. The results of isolation and purification by HPLC and analysis by mass spectrometry are shown in Figure 6 and Figure 11. The theoretical molecular weight of ADMR-011 polypeptide was 4258.7 Da, and its molecular weight analyzed by mass spectrometry was 4258.8 Da, which was identical to the molecular weight deduced according to the amino acid sequence.

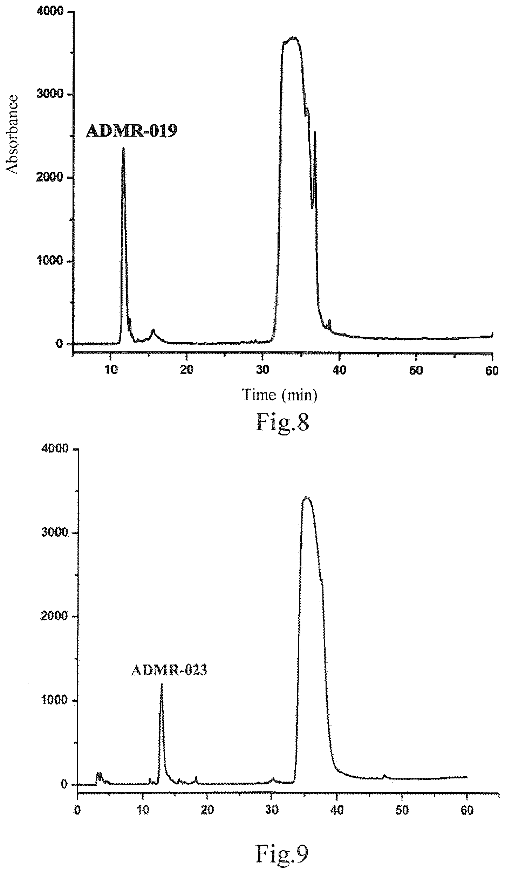

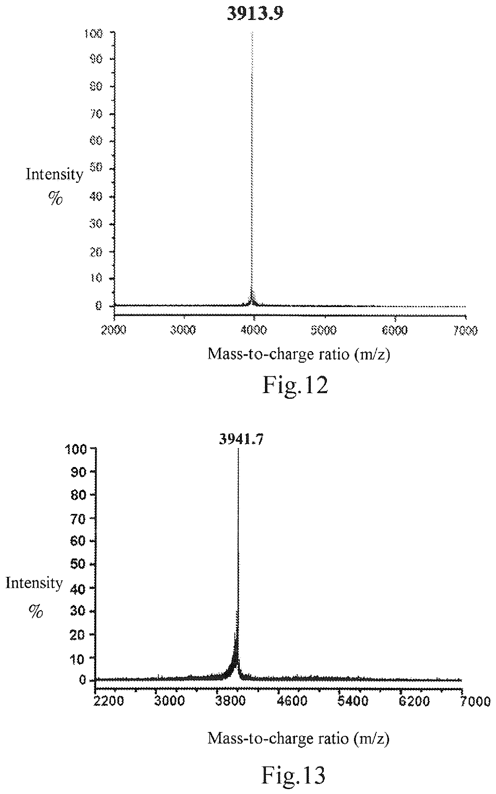

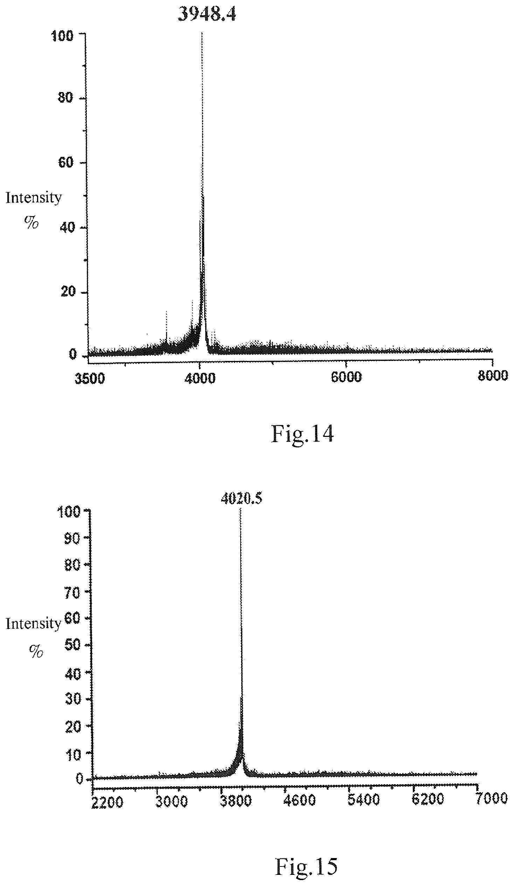

The process and steps for expressing and purifying scorpion active polypeptides ADMR-016, ADMR-019, ADMR-023 and ADMR-028 were the same to that of ADMR-011, and the results for isolation and purification and mass spectroscopic analysis of related polypeptides are shown in Figure 2, Figure 7, Figure 12; Figure 3, Figure 8, Figure 13; Figure 4, Figure 9, Figure 14; Figure 5, Figure 10, Figure 15, respectively. The theoretical molecular weights of ADMR-016, ADMR-019, ADMR-023 and ADMR-028 polypeptides were 3912.6Da, 3940.6Da, 3948.6 Da, and 4020.7 Da, respectively, and their molecular weights analyzed by mass spectrometry were 3912.9 Da, 3940.7Da, 3948.4 Da, and 4020.5Da, respectively, which were identical to the molecular weights deduced according to the amino acid sequences.

### Example 4: Analysis of pharmacological activities of scorpion active peptides ADMR-011, ADMR-016, ADMR-019, ADMR-023 and ADMR-028 on potassium channel Kv1.3

COS-7 cells were cultured in a DMEM medium containing 10% fetal calf serum, at 37° C, 5% CO₂. The recombinant plasmids of potassium channel Kv1.3 were transfected by using a Sofast™ transfection kit, and the transfected cells were selectively cultured in a medium containing 0.8 mg/mL geneticin. The pharmacological activities of scorpion active polypeptides ADMR-011, ADMR-016, ADMR-019, ADMR-023 and ADMR-028 were tested and analyzed by using an instrument of whole-cell patch clamp (EPC-10 dual-channel patch clamp amplifier HEKA, Elektronik, Lambrecht, Germany). The setting of the experimental parameters, the collecting of data, and the applying of stimulus were all controlled by Pulse software (Elektronik, Lambrecht, Germany). The filter of the instrument was set to be 10 kHz (Bessel), and the electrode impedance was 2∼5 MΩ. After the formation of giga-seal (1∼5 GΩ) between the electrode and cell membrane, an automatic c-fast was performed, and the membrane was broken by slightly applying negative pressure, then an automatic c-slow was performed. At a holding potential of -70 mV, depolarizing pulses, incrementing in 10 mV steps, with a step width of 80 ms , were given to stimulate from -60 mV to +50 mV, and the condition of current was observed. The polypeptides ADMR-011, ADMR-016 and ADMR-019 were accurately perfused, which was achieved by MPS-2 (INBIO Inc, Wuhan, China) perfusing system. After dissolving the three scorpion active polypeptides, they were applied by spraying via DAD Drug Application System (ALA), and the tip of the tube for drug application was about 100 µm away from the cell being recorded. The results are shown in Figure 11 to Figure 15.

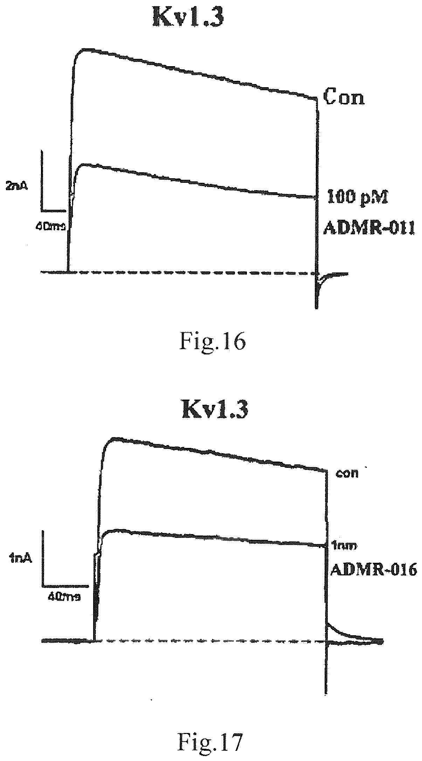

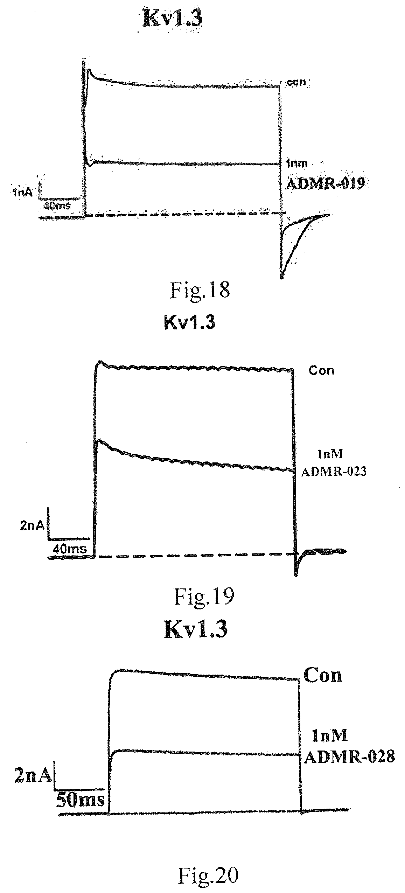

It can be known from Figure 16 to Figure 20 that, the pharmacological activity of ADMR-011 on potassium channel Kvl.3 was 91 pM, the pharmacological activity of ADMR-016 on potassium channel Kvl.3 was 2.58 nM, the pharmacological activity of ADMR-019 on potassium channel Kv1.3 was 1.41 nM, and the pharmacological activity of ADMR-023 and ADMR-028 on potassium channel Kvl.3 were 0.49 nM and 0.65 nM, respectively. Therefore, they are polypeptides with potent activity that are currently found internationally.

### Example 5: Pharmacodynamic experiments for treating multiple sclerosis by scorpion active polypeptides ADMR-011, ADMR-016, ADMR-019, ADMR-023 and ADMR-028

Experimental animals: female inbred line Wistar rats (6∼8 week old, body weigh 150±10 g) and guinea pigs (300∼400 g, purchased from Wuhan University Laboratory Animal Center) were selected.

Main reagents: Freund's complete adjuvant (Gibcol/BRL), BCG vaccine, pertussis vaccine (Shanghai Institute of Biological Products), and guinea pig MBP (Sigma).

Formulating a mixed emulsion of whole spinal cord homogenate - Freund's complete adjuvant (GPSCH-CFA): a guinea pig was executed, and the spinal cord was rapidly removed, and prepared into 50% PBS homogenate by a ultrasonic disruptor (Sonics & Materials Inc, America), which was mixed with equal amount of Freund's complete adjuvant (BCG vaccine 10 mg/ml), and was pushed back and forth with syringes until forming a water-in-oil emulsion.

Wistar rat EAE model induced by EAE: GPSCH-CFA emulsion was injected intradermally into the foot pads of both the hind legs of Wistar rats, or simultaneously injected intradermally about 1×10¹⁰ pertussis vaccine. The rats were weighed every day, and observed for their neurological symptoms. Experimental autoimmune encephalomyelitis appeared after the rats had been raised for 2 weeks.

The rats with experimental autoimmune encephalomyelitis (EAE) were divided into five groups randomly: normal control group (negative control group), model control group (model rats + normal saline), administration group A (model rats + ADMR-011 polypeptide), administration group B (model rats + ADMR-016 polypeptide), administration group C (model rats + ADMR-019 polypeptide), administration group D (model rats + ADMR-023 polypeptide), and administration group E (model rats + ADMR-028 polypeptide), 10 rats for each group. For the administration groups, polypeptides ADMR-011, ADMR-016, ADMR-019, ADMR-023 and ADMR-028 were injected subcutaneously respectively at a dose of 100 µg·kg⁻¹, once every day in the morning. For the normal control group and the model control group, equivalent amount of normal saline was injected subcutaneously, and administered continuously. After administered for 14 days, the rats were observed for their conditions of suffering from autoimmune encephalomyelitis, and scored according to the conditions. The highest clinical score for each animal was recorded, and the average value of them was taken to be the average clinical score. The results are shown in Table 1.

The scoring criteria were: without any clinical symptom, 0 point; disappearance of tension in the tail, and observable mild awkward gait, 1 point; weakness in both the hind limbs, which can recover after passive body flipping, 2 points; paralysis in both the hind limbs, which can not recover after passive body flipping, 3 points; tetraplegia accompanied by incontinence of urine and feces, 4 points; moribund state or death, 5 points.

**Table 1. Scores for rats of various experimental groups in the EAE model (⁻x±s)**

| Groups | n | Average clinical scores | | | |
|---|---|---|---|---|---|
| | | 8 d | 10 d | 12 d | 14 d |
| Normal control group | 10 | 0.00±0.00 | 0.00±0.00 | 0.00±0.00 | 0.00±0.00 |
| Model control group | 10 | 0.51±0.19 | 1.29±0.48 | 3.38±0.39 | 2.93±0.16 |
| Administration group A | 10 | 0.47±0.18 | 0.75±0.21 | 0.83±0.35 | 0.95±0.37 |
| Administration group B | 10 | 0.50±0.23 | 0.87±0.34 | 1.31±0.42 | 1.21±0.49 |
| Administration group C | 10 | 0.53±0.16 | 0.93±0.46 | 1.19±0.51 | 1.37±0.34 |
| Administration group D | 10 | 0.55±0.19 | 0.81±0.29 | 1.21±0.25 | 1.17±0.27 |
| Administration group E | 10 | 0.49±0.12 | 0.95±0.22 | 1.09±0.37 | 1.13±0.29 |

Experimental results showed that, in the absence of medication, the score in the model control group was the highest (2.93 ± 0.16); after treating by scorpion active polypeptides ADMR-011, ADMR-016, ADMR-019, ADMR-023 and ADMR-028, the symptoms of rats were improved significantly, and the average clinical scores were 0.95±0.37, 1.21±0.49, 1.37±0.34, 1.17±0.27, and 1.13±0.29, respectively. Thus, it can be seen that scorpion active polypeptides ADMR-011, ADMR-016, ADMR-019, ADMR-023 and ADMR-028 can effectively treat multiple sclerosis.

### Example 6: Pharmacodynamic experiments for treating rheumatoid arthritis by scorpion active polypeptides ADMR-011, ADMR-016, ADMR-019, ADMR-023 and ADMR-028

40 Female (♀) specific pathogen free (SPF) inbred line SD rats (Wuhan University Laboratory Animal Center), body weight 150±10 g, were selected, and pristane was injected intradermally at the base of the tail of rats in experimental groups at a dose of 0.2 ml/rat, after they were acclimatized in a clean-grade environment for 1 week. Rheumatoid arthritis appeared after the rats were raised for only 2 weeks.

The rats having arthritis were randomized into: normal control group (negative control group), model negative control group (model rat + normal saline), model positive control group (model rat + methotrexate), administration group A (model rat + ADMR-011 polypeptide), administration group B (model rat + ADMR-016 polypeptide), administration group C (model rat + ADMR-019 polypeptide), administration group D (model rat + ADMR-023 polypeptide), and administration group E (model rat + ADMR-028 polypeptide), 10 rats for each group. The administration groups were injected subcutaneously at a dose of 100 µg·kg⁻¹, once every day in the morning, and administered continuously; the normal control group and the model negative control group were injected subcutaneously with equivalent amount of normal saline; and the model positive control group were injected subcutaneously with methotrexate (MTX) at a dose of 1.75 µg·kg⁻¹, once every day, and administered continuously.

After administered for 21 days, the rats were observed for their conditions of suffering from rheumatoid arthritis, and scored according to the conditions. The highest clinical score for each animal was recorded, and the average value of them was taken to be the average clinical score. The results are shown in Table 2. The scoring criteria were: one joint of a rat was red and swollen, 1 point; two joints of a rat were red and swollen, 2 points; all the joints of a rat were red and swollen, 3 points; severe arthritis in the entire limb of a rat, 4 points.

**Table 2. Arthritis scores for rats of various experimental groups (⁻x±s)**

| Groups | n | Score of joint | | |
|---|---|---|---|---|
| | | 10 d | 15 d | 21 d |
| Normal control group | 10 | 0.00±0.00 | 0.00±0.00 | 0.00±0.00 |
| Model negative control group | 10 | 2.07±0.63 | 2.64±0.76 | 3.67±0.85 |
| Model positive control group | 10 | 1.73±0.67 | 1.96±0.73 | 1.67±0.82 |
| Administration group A | 10 | 1.63±0.57 | 2.07±0.65 | 1.62±0.59 |
| Administration group B | 10 | 1.84±0.71 | 2.24±0.53 | 1.96±0.62 |
| Administration group C | 10 | 1.78±0.48 | 2.17±0.55 | 1.81±0.77 |
| Administration group D | 10 | 1.69±0.23 | 2.23±0.71 | 1.85±0.59 |
| Administration group E | 10 | 1.79±0.55 | 2.11±0.45 | 1.79±0.63 |

From day 8 of the experiment, the hind foot plantar volume of the rat was measured and recorded every other day for 21 consecutive days, and the swelling degree (mL) was calculated according to the volume difference before and after the modeling. The results are shown in Table 3.

**Table 3. The swelling degree of rats in various experimental groups (⁻x±s)**

| Groups | n | Foot volume before the inflammation (mL) | The swelling degree after the inflammation (mL) | | | |
|---|---|---|---|---|---|---|
| | | | d8 | d15 | d22 | d28 |
| Normal control group | 10 | 1.13±0.11 | 0.038±0.011 | 0.09±0.01 | 0.10±0.02 | 0.12±0.2 |
| Model negative control group | 10 | 1.15±0.12 | 0.037±0.010 | 0.32±0.05 | 0.53±0.12 | 0.51±0.11 |
| Model positive control group | 10 | 1.14±0.11 | 0.039±0.013 | 0.20±0.01 | 0.31±0.14 | 0.22±0.12 |
| Administration group A | 10 | 1.13±0.14 | 0.041±0.012 | 0.21±0.06 | 0.29±0.09 | 0.24±0.13 |
| Administration group B | 10 | 1.16 ±0.09 | 0.037±0.012 | 0.25±0.07 | 0.37±0.08 | 0.33±0.12 |
| Administration group C | 10 | 1.14 ±0. 12 | 0.034±0.011 | 0.27±0.07 | 0.34±0.11 | 0.37±0.13 |
| Administration group D | 10 | 1.15±0.10 | 0.035±0.010 | 0.25±0.08 | 0.32±0.14 | 0.29±0.15 |
| Administration group E | 10 | 1.14±0.15 | 0.037±0.013 | 0.28±0.05 | 0.38±0.13 | 0.31±0.14 |

It can be known from Table 2 that, in the absence of medication, the score of joint in the model negative control group is the highest 3.67 ± 0.85); after treating by scorpion active polypeptides ADMR-011, ADMR-016, ADMR-019, ADMR-023 and ADMR-028, the symptoms of rats are improved significantly, and the average clinical scores are 1.62±0.59, 1.96±0.62, 1.81±0.77, 1.85±0.59, and 1.79±0.63, respectively, which are similar to the results of the methotrexate treatment group (1.67±0.82). The results of foot plantar swelling degree of rats in various experimental groups measured in Table 3 show that the foot plantar swelling degrees of rats in 3 scorpion active polypeptides administration groups are significantly reduced comparing to the model negative control group. The results suggest that scorpion active polypeptides ADMR-011, ADMR-016, ADMR-019, ADMR-023 and ADMR-028 can effectively treat rheumatoid arthritis.

### Example 7: Toxicity study of scorpion active polypeptides ADMR-011, ADMR-016, ADMR-019, ADMR-023 and ADMR-028

18∼20 g Kunming mice were selected, and divided into 4 groups, 8 male mice and 8 female mice for each group. The lyophilized powders of scorpion active polypeptides ADMR-011, ADMR-016, ADMR-019, ADMR-023 and ADMR-028 were dissolved with normal saline respectively, and the 3 groups of mice were injected intraperitoneally once respectively with polypeptide solution at a dose of 10 mg/kg (100 times of the dose for animal model), and the fourth group were injected with equivalent amount of normal saline as control. The mice were observed for 7 consecutive days, and the effects of the drugs on the respiratory center, cardio-pulmonary function and central nervous system (CNS) of the animals after the administration were evaluated.

After the administration, the respiration, motor and heartbeat in animals of all the groups were all normal, without significant abnormal reaction. 7 days after the administration, all the animals were conducted gross anatomy to observe the volume, color and texture of the main organs (heart, liver, spleen, lung, and kidney), and there was no macroscopic lesion. There were no significant difference between the 3 administration groups and the normal saline group. The experimental results suggested that the scorpion active polypeptides ADMR-011, ADMR-016, ADMR-019, ADMR-023 and ADMR-028 had no significant toxicity in mice at a dose 100 times more than the therapeutic dose for the animal model of an autoimmune disease.

## Claims

1. An α-KTX family scorpion active polypeptide which acts on potassium channel Kvl.3, **characterized in that** the polypeptide has the amino acid sequence as set forth in SEQ ID NO: 1, SEQ ID NO: 2, SEQ ID NO: 3, SEQ ID NO: 13 or SEQ ID NO: 14.

2. A DNA molecule encoding the scorpion active polypeptide according to claim 1.

3. A DNA molecule encoding the amino acid sequence as set forth in SEQ ID NO: 1, **characterized in that** the DNA molecule has the nucleotide sequence as set forth in SEQ ID NO: 4; or
a DNA molecule encoding the amino acid sequence as set forth in SEQ ID NO: 2, **characterized in that** the DNA molecule has the nucleotide sequence as set forth in SEQ ID NO: 5; or
a DNA molecule encoding the amino acid sequence as set forth in SEQ ID NO: 3, **characterized in that** the DNA molecule has the nucleotide sequence as set forth in SEQ ID NO: 6; or
a DNA molecule encoding the amino acid sequence as set forth in SEQ ID NO: 13, **characterized in that** the DNA molecule has the nucleotide sequence as set forth in SEQ ID NO: 15; or
a DNA molecule encoding the amino acid sequence as set forth in SEQ ID NO: 14, **characterized in that** the DNA molecule has the nucleotide sequence as set forth in SEQ ID NO: 16.

4. A vector having the DNA molecule according to claim 2 or 3.

5. The vector according to claim 4, **characterized in that** the vector is pGEX-6p-1.

6. The vector according to claim 5, **characterized in that** the vector has the nucleotide sequence as set forth in SEQ ID NO: 4, SEQ ID NO: 5 or SEQ ID NO: 6.

7. A method for preparing the scorpion active polypeptide according to claim 1, comprising:
step 1: obtaining a DNA molecule that can encode the amino acid sequence as set forth in SEQ ID NO: 1, SEQ ID NO: 2, SEQ ID NO: 3, SEQ ID NO: 13 or SEQ ID NO: 14;
step 2: fusing the DNA molecule obtained in step 1 with an expression vector, and constructing a recombinant expression vector, and transforming a host cell;
step 3: inducing the host cell containing the recombinant expression vector to express the protein, and isolating and purifying the expressed protein.

8. The preparation method according to claim 7, **characterized in that** step 1 is specifically carried out by an amplification using a template of cDNA encoding the amino acid sequence as set forth in SEQ ID NO: 1, and an upstream primer having the nucleotide sequence as set forth in SEQ ID NO: 7 and a downstream primer having the nucleotide sequence as set forth in SEQ ID NO: 8; or
**characterized in that** step 1 is specifically carried out by an amplification using a template of cDNA encoding the amino acid sequence as set forth in SEQ ID NO: 2, and an upstream primer having the nucleotide sequence as set forth in SEQ ID NO: 9 and a downstream primer having the nucleotide sequence as set forth in SEQ ID NO: 10; or
**characterized in that** step 1 is specifically carried out by an amplification using a template of cDNA encoding the amino acid sequence as set forth in SEQ ID NO: 3, and an upstream primer having the nucleotide sequence as set forth in SEQ ID NO: 11 and a downstream primer having the nucleotide sequence as set forth in SEQ ID NO: 12.; or
**characterized in that** step 1 is specifically carried out by an amplification using a template of cDNA encoding the amino acid sequence as set forth in SEQ ID NO: 13, and an upstream primer having the nucleotide sequence as set forth in SEQ ID NO: 17 and a downstream primer having the nucleotide sequence as set forth in SEQ ID NO: 18; or
**characterized in that** step 1 is specifically carried out by an amplification using a template of cDNA encoding the amino acid sequence as set forth in SEQ ID NO: 14, and an upstream primer having the nucleotide sequence as set forth in SEQ ID NO: 19 and a downstream primer having the nucleotide sequence as set forth in SEQ ID NO: 20.

9. The preparation method according to claim 7, **characterized in that** the expression vector of step 2 is pGEX-6p-1.

10. The preparation method according to claim 7, **characterized in that** the host cell is of Rosetta (DE3) strain.

11. A scorpion active polypeptide according to claim 1 for use in treating or preventing a disease associated with potassium channel Kv1.3, wherein the disease associated with Kv1.3 is an autoimmune disease.

12. Use of the scorpion active polypeptide according to claim 1 in the manufacture of a medicament for treating or preventing a disease associated with potassium channel Kv1.3, wherein the disease associated with Kv1.3 is an autoimmune disease.

13. A scorpion active polypeptide for use in treating or preventing a disease associated with potassium channel Kv1.3 according to claim 11, **characterized in that** the autoimmune disease is multiple sclerosis or rheumatoid arthritis.

14. The use of a scorpion active polypeptide according to claim 12, **characterized in that** the autoimmune disease is multiple sclerosis or rheumatoid arthritis.

## Patentansprüche

1. Skorpionaktives Polypeptid der α-KTX-Familie, das auf den Kaliumkanal Kv1.3 wirkt,
**dadurch gekennzeichnet, dass** das Polypeptid die in SEQ ID NO: 1, SEQ ID NO: 2, SEQ ID NO: 3, SEQ ID NO: 13 oder SEQ ID NO: 14 aufgeführte Aminosäuresequenz aufweist.

2. DNA-Molekül, welches für das skorpionaktive Polypeptid nach Anspruch 1 codiert.

3. DNA-Molekül, welches für die in SEQ ID NO: 1 aufgeführte Aminosäuresequenz codiert, **dadurch gekennzeichnet, dass** das DNA-Molekül die in SEQ ID NO: 4 aufgeführte Nukleotidsequenz aufweist; oder
DNA-Molekül, welches für die in SEQ ID NO: 2 aufgeführte Aminosäuresequenz codiert, **dadurch gekennzeichnet, dass** das DNA-Molekül die in SEQ ID NO: 5 aufgeführte Nukleotidsequenz aufweist; oder
DNA-Molekül, welches für die in SEQ ID NO: 3 aufgeführte Aminosäuresequenz codiert, **dadurch gekennzeichnet, dass** das DNA-Molekül die in SEQ ID NO: 6 aufgeführte Nukleotidsequenz aufweist; oder
DNA-Molekül, welches für die in SEQ ID NO: 13 aufgeführte Aminosäuresequenz codiert, **dadurch gekennzeichnet, dass** das DNA-Molekül die in SEQ ID NO: 15 aufgeführte Nukleotidsequenz aufweist; oder
DNA-Molekül, welches für die in SEQ ID NO: 14 aufgeführte Aminosäuresequenz codiert, **dadurch gekennzeichnet, dass** das DNA-Molekül die in SEQ ID NO: 16 aufgeführte Nukleotidsequenz aufweist.

4. Vektor, welcher das DNA-Molekül nach Anspruch 2 oder 3 aufweist.

5. Vektor nach Anspruch 4, **dadurch gekennzeichnet, dass** der Vektor pGEX-6p-1 ist.

6. Vektor nach Anspruch 5, **dadurch gekennzeichnet, dass** der Vektor die in SEQ ID NO: 4, SEQ ID NO: 5 oder SEQ ID NO: 6 aufgeführte Nukleotidsequenz aufweist.

7. Verfahren zum Herstellen des skorpionaktiven Polypeptids nach Anspruch 1, umfassend:
Schritt 1: Erhalten eines DNA-Moleküls, welches für die in SEQ ID NO: 1, SEQ ID NO: 2, SEQ ID NO: 3, SEQ ID NO: 13 oder SEQ ID NO: 14 aufgeführte Aminosäuresequenz codieren kann;
Schritt 2: Fusionieren des in Schritt 1 erhaltenen DNA-Moleküls mit einem Expressionsvektor und Konstruieren eines rekombinanten Expressionsvektors, und Transformieren einer Wirtszelle;
Schritt 3: Induzieren der den rekombinanten Expressionsvektor enthaltenden Wirtszelle, um das Protein zu exprimieren, und Isolieren und Reinigen des exprimierten Proteins.

8. Herstellverfahren nach Anspruch 7, **dadurch gekennzeichnet, dass** Schritt 1 gezielt durch ein Amplifizieren unter Verwendung einer Matrize von cDNA, welche für die in SEQ ID NO: 1 aufgeführte Aminosäuresequenz codiert, und eines stromaufwärtigen Primers mit der in SEQ ID NO: 7 aufgeführten Nukleotidsequenz und eines stromabwärtigen Primers mit der in SEQ ID NO: 8 aufgeführten Nukleotidsequenz ausgeführt wird; oder
**dadurch gekennzeichnet, dass** Schritt 1 gezielt durch ein Amplifizieren unter Verwendung einer Matrize von cDNA, welche für die in SEQ ID NO: 2 aufgeführte Aminosäuresequenz codiert, und eines stromaufwärtigen Primers mit der in SEQ ID NO: 9 aufgeführten Nukleotidsequenz und eines stromabwärtigen Primers mit der in SEQ ID NO: 10 aufgeführten Nukleotidsequenz ausgeführt wird; oder
**dadurch gekennzeichnet, dass** Schritt 1 gezielt durch ein Amplifizieren unter Verwendung einer Matrize von cDNA, welche für die in SEQ ID NO: 3 aufgeführte Aminosäuresequenz codiert, und eines stromaufwärtigen Primers mit der in SEQ ID NO: 11 aufgeführten Nukleotidsequenz und eines stromabwärtigen Primers mit der in SEQ ID NO: 12 aufgeführten Nukleotidsequenz ausgeführt wird; oder
**dadurch gekennzeichnet, dass** Schritt 1 gezielt durch ein Amplifizieren unter Verwendung einer Matrize von cDNA, welche für die in SEQ ID NO: 13 aufgeführte Aminosäuresequenz codiert, und eines stromaufwärtigen Primers mit der in SEQ ID NO: 17 aufgeführten Nukleotidsequenz und eines stromabwärtigen Primers mit der in SEQ ID NO: 18 aufgeführten Nukleotidsequenz ausgeführt wird; oder
**dadurch gekennzeichnet, dass** Schritt 1 gezielt durch ein Amplifizieren unter Verwendung einer Matrize von cDNA, welche für die in SEQ ID NO: 14 aufgeführte Aminosäuresequenz codiert, und eines stromaufwärtigen Primers mit der in SEQ ID NO: 19 aufgeführten Nukleotidsequenz und eines stromabwärtigen Primers mit der in SEQ ID NO: 20 aufgeführten Nukleotidsequenz ausgeführt wird.

9. Herstellverfahren nach Anspruch 7, **dadurch gekennzeichnet, dass** der Expressionsvektor von Schritt 2 pGEX-6p-1 ist.

10. Herstellverfahren nach Anspruch 7, **dadurch gekennzeichnet, dass** die Wirtszelle vom Rosetta(DE3)-Stamm ist.

11. Skorpionaktives Polypeptid nach Anspruch 1 zur Verwendung beim Behandeln oder Verhindern einer mit dem Kaliumkanal Kv1.3 in Zusammenhang stehenden Erkrankung, wobei die mit Kv1.3 in Zusammenhang stehende Erkrankung eine Autoimmunerkrankung ist.

12. Verwendung des skorpionaktiven Polypeptids nach Anspruch 1 beim Herstellen eines Medikaments zum Behandeln oder Verhindern einer mit dem Kaliumkanal Kv1.3 in Zusammenhang stehenden Erkrankung, wobei die mit Kv1.3 in Zusammenhang stehende Erkrankung eine Autoimmunerkrankung ist.

13. Skorpionaktives Polypeptid zur Verwendung beim Behandeln oder Verhindern einer mit dem Kaliumkanal Kv1.3 in Zusammenhang stehenden Erkrankung nach Anspruch 11, **dadurch gekennzeichnet, dass** die Autoimmunerkrankung multiple Sklerose oder rheumatoide Arthritis ist.

14. Verwendung eines skorpionaktiven Polypeptids nach Anspruch 12, **dadurch gekennzeichnet, dass** die Autoimmunerkrankung multiple Sklerose oder rheumatoide Arthritis ist.

## Revendications

1. Polypeptide de scorpion actif de la famille des α-KTX qui agit sur le canal potassique Kv1.3, **caractérisé en ce que** le polypeptide présente la séquence d'acides aminés décrite par la séquence SEQ ID N° : 1, SEQ ID N° : 2, SEQ ID N° : 3, SEQ ID N° : 13 ou SEQ ID N° : 14.

2. Molécule d'ADN codant pour le polypeptide de scorpion actif selon la revendication 1.

3. Molécule d'ADN codant pour la séquence d'acides aminés décrite par la séquence SEQ ID N° : 1, **caractérisée en ce que** la molécule d'ADN présente la séquence nucléotidique décrite par la séquence SEQ ID N° : 4 ; ou
molécule d'ADN codant pour la séquence d'acides aminés décrite par la séquence SEQ ID N° : 2, **caractérisée en ce que** la molécule d'ADN présente la séquence nucléotidique décrite par la séquence SEQ ID N° : 5 ; ou
molécule d'ADN codant pour la séquence d'acides aminés décrite par la séquence SEQ ID N° : 3, **caractérisée en ce que** la molécule d'ADN présente la séquence nucléotidique décrite par la séquence SEQ ID N° : 6 ; ou
molécule d'ADN codant pour la séquence d'acides aminés décrite par la séquence SEQ ID N° : 13, **caractérisée en ce que** la molécule d'ADN présente la séquence nucléotidique décrite par la séquence SEQ ID N° : 15 ; ou
molécule d'ADN codant pour la séquence d'acides aminés décrite par la séquence SEQ ID N° : 14, **caractérisée en ce que** la molécule d'ADN présente la séquence nucléotidique décrite par la séquence SEQ ID N° : 16.

4. Vecteur ayant la molécule d'ADN selon la revendication 2 ou 3.

5. Vecteur selon la revendication 4, **caractérisé en ce que** le vecteur est pGEX-6p-1.

6. Vecteur selon la revendication 5, **caractérisé en ce que** le vecteur présente la séquence nucléotidique décrite par la séquence SEQ ID N° : 4, SEQ ID N° : 5 ou SEQ ID N° : 6.

7. Procédé de préparation du polypeptide de scorpion actif selon la revendication 1, comprenant :
étape 1 : obtention d'une molécule d'ADN qui peut coder pour la séquence d'acides aminés décrite par la séquence SEQ ID N° : 1, SEQ ID N° : 2, SEQ ID N° : 3, SEQ ID N° : 13 ou SEQ ID N° : 14 ;
étape 2 : fusion de la molécule d'ADN obtenue à l'étape 1 avec un vecteur d'expression, construction d'un vecteur d'expression recombinant et transformation d'une cellule hôte ;
étape 3 : induction de la cellule hôte contenant le vecteur d'expression recombinant pour qu'elle exprime la protéine et isolation et purification de la protéine exprimée.

8. Procédé de préparation selon la revendication 7, **caractérisé en ce que** l'étape 1 est spécifiquement effectuée par une amplification en utilisant une matrice d'ADNc qui code pour la séquence d'acides aminés décrite par la séquence SEQ ID N° : 1, une amorce amont ayant la séquence nucléotidique décrite par la séquence SEQ ID N° : 7 et une amorce aval ayant la séquence nucléotidique décrite par la séquence SEQ ID N° : 8 ; ou
**caractérisé en ce que** l'étape 1 est spécifiquement effectuée par une amplification en utilisant une matrice d'ADNc qui code pour la séquence d'acides aminés décrite par la séquence SEQ ID N° : 2, une amorce amont ayant la séquence nucléotidique décrite par la séquence SEQ ID N° : 9 et une amorce aval ayant la séquence nucléotidique décrite par la séquence SEQ ID N° : 10 ; ou
**caractérisé en ce que** l'étape 1 est spécifiquement effectuée par une amplification en utilisant une matrice d'ADNc qui code pour la séquence d'acides aminés décrite par la séquence SEQ ID N° : 3, une amorce amont ayant la séquence nucléotidique décrite par la séquence SEQ ID N° : 11 et une amorce aval ayant la séquence nucléotidique décrite par la séquence SEQ ID N° : 12 ; ou
**caractérisé en ce que** l'étape 1 est spécifiquement effectuée par une amplification en utilisant une matrice d'ADNc qui code pour la séquence d'acides aminés décrite par la séquence SEQ ID N° : 13, une amorce amont ayant la séquence nucléotidique décrite par la séquence SEQ ID N° : 17 et une amorce aval ayant la séquence nucléotidique décrite par la séquence SEQ ID N° : 18 ; ou
**caractérisé en ce que** l'étape 1 est spécifiquement effectuée par une amplification en utilisant une matrice d'ADNc qui code pour la séquence d'acides aminés décrite par la séquence SEQ ID N° : 14, une amorce amont ayant la séquence nucléotidique décrite par la séquence SEQ ID N° : 19 et une amorce aval ayant la séquence nucléotidique décrite par la séquence SEQ ID N° : 20.

9. Procédé de préparation selon la revendication 7, **caractérisé en ce que** le vecteur d'expression de l'étape 2 est pGEX-6p-1.

10. Procédé de préparation selon la revendication 7, **caractérisé en ce que** la cellule hôte est de la souche Rosetta (DE3).

11. Polypeptide de scorpion actif selon la revendication 1 pour une utilisation dans le traitement ou la prévention d'une maladie associée au canal potassique Kv1.3, où la maladie associée au canal potassique Kv1.3 est une maladie auto-immune.

12. Utilisation du polypeptide de scorpion actif selon la revendication 1 dans la fabrication d'un médicament pour le traitement ou la prévention d'une maladie associée au canal potassique Kv1.3, où la maladie associée au canal potassique Kv1.3 est une maladie auto-immune.

13. Polypeptide de scorpion actif pour une utilisation dans le traitement ou la prévention d'une maladie associée au canal potassique Kv1.3 selon la revendication 11, **caractérisé en ce que** la maladie auto-immune est la sclérose en plaques ou la polyarthrite rhumatoïde.

14. Utilisation d'un polypeptide de scorpion actif selon la revendication 12, **caractérisée en ce que** la maladie auto-immune est la sclérose en plaques ou la polyarthrite rhumatoïde.
